# EUROPEAN PATENT APPLICATION

(11) **EP 4 835 090 A1**
(43) Date of publication of application: **04.11.2026**
(21) Application number: 25745354.8
(22) Date of filing: 23.01.2025
(51) Int. Cl.: C12P 7/44, C07C 51/43, C07C 63/26

(54) **PROCESS FOR EFFICIENTLY COLLECTING TEREPHTHALIC ACID (TPA) IN ENZYMATIC DEGRADATION PROCESS OF POLYESTER**

(30) Priority: 24.01.2024 KR 20240010912
(71) Applicant: CJ Cheiljedang Corporation, Seoul 04560 (KR)
(72) Inventor: LEE, In Sung, Seoul 04560 (KR); KIM, Hyung Joon, Seoul 04560 (KR); SEO, Yong Bum, Seoul 04560 (KR); JANG, Jaewon, Seoul 04560 (KR); KIM, Min Sup, Seoul 04560 (KR); RYU, Ae Jin, Seoul 04560 (KR); KIM, Gyeonghwan, Seoul 04560 (KR)
(74) Representative: Meissner Bolte Partnerschaft mbB
(86) International application number: PCT/KR2025/001324
(87) International publication number: WO 2025/159530

(57) **Abstract**

The present disclosure relates to a process for efficiently recovering terephthalic acid in a polyester enzymatic degradation process, wherein the polyester comprises terephthalic acid as a monomer.

## Description

### [Technical Field]

The present disclosure relates to a process for efficiently recovering terephthalic acid (TPA) in a polyester enzymatic degradation process, wherein the polyester comprises terephthalic acid as a monomer.

### [Background Art]

Plastics are newly produced in an amount of more than 400 million tons every year, and as environmental issues regarding waste plastics have been highlighted, efforts are being made to reduce production through regulation of disposable products and use of plastic alternatives, but in reality, production is increasing every year.

Polyethylene terephthalate (PET), which accounts for less than 10% of all plastics, is newly produced in an amount of approximately 360 million tons every year, and because it is mainly used for disposable products, it is considered to be a plastic having the shortest life cycle. Recycling of waste plastics includes mechanical recycling, pyrolysis, chemical recycling, and the like, and each method is entering commercialization or a final research stage for commercialization. Each technology may be a solution to waste plastic issues, but none of the existing methods can be considered perfect due to impacts on quality deterioration caused by downcycling, carbon neutrality, resource depletion, eutrophication of seawater and freshwater, and the like.

In order to solve environmental problems caused by waste plastics, such as microplastics, greenhouse gas generation, and resource depletion, a series of research results have been published regarding degradation of PET, which is one type of representative plastic, by biological technology using enzymes, and unlike conventional recycling methods, an advantage of these enzymatic methods is that they make it possible to recover terephthalic acid (TPA) and ethylene glycol (EG) from PET, and allow them to be used for making new polymers, thereby enabling infinite recycling. However, a process for recovering, at a high yield, high-purity TPA that can be repolymerized into PET from a reaction solution after degradation is still complicated and difficult, and becomes an obstacle to securing the economic feasibility of technology for recycling waste plastics through biological degradation of PET.

### [Prior Art Documents]

(Patent Document 1) KR 10-2021-0091202 A
(Patent Document 2) EP 3877458 A1
(Patent Document 3) US 2022-0002516 A1

### [Disclosure]

### [Technical Problem]

The present disclosure relates to a process for efficiently recovering terephthalic acid in a polyester enzymatic degradation process, wherein the polyester comprises terephthalic acid as a monomer.

### [Technical Solution]

An object of the present disclosure is to provide a method for preparing terephthalic acid, the method comprising depolymerizing a polyester comprising terephthalic acid as a monomer with an enzyme while maintaining the pH in a reactor at 5 to 9; and, after completion of an enzymatic reaction, adding a base to the reaction mixture to maintain pH at 9.0 or more.

### [Advantageous Effects]

Using the method of the present disclosure, the yield of terephthalic acid produced by enzymatic degradation of a polyester comprising terephthalic acid as a monomer may be increased.

### [Detailed Description of Preferred Embodiments]

The present disclosure will be described in detail below. Meanwhile, each description and embodiment disclosed in the present disclosure may also be applied to each other description and embodiment. That is, all combinations of various elements disclosed in the present disclosure fall within the scope of the present disclosure. In addition, the scope of the present disclosure cannot be considered to be limited by the specific description provided below.

In addition, those skilled in the art can recognize or confirm numerous equivalents to specific aspects of the present disclosure described herein using only routine experimentation. In addition, such equivalents are intended to be included in the present disclosure.

In addition, numerous papers and patent documents are referenced throughout this specification, and citations thereof are indicated. The disclosures of the cited papers and patent documents are incorporated herein by reference in their entirety, whereby the level of the technical field to which the present disclosure belongs and the contents of the present disclosure are more clearly described.

One aspect of the present disclosure is a method for preparing terephthalic acid, the method comprising depolymerizing a polyester comprising terephthalic acid as a monomer with an enzyme; and adding a base to the reaction mixture to maintain pH at 9.0 or more.

As used herein, the term "polyester" refers to a polymer comprising an ester functional group in the main chain of its structure. For purposes of the present disclosure, the polyester is a polyester comprising terephthalic acid as a monomer.

In one embodiment, the polyester may be selected from polyethylene terephthalate, polyethylene terephthalate glycol (PETG), polyethylene co-isosorbide terephthalate (PEIT), polytrimethylene terephthalate (PTT), polybutylene adipate terephthalate (PBAT), polycyclohexylenedimethylene terephthalate (PCT), and polybutylene terephthalate (PBT).

The polyethylene terephthalate of the present disclosure is a semi-aromatic copolymer consisting of two monomers, terephthalic acid and ethylene glycol. Terephthalic acid is produced by depolymerization of polyethylene terephthalate.

In the method of the present disclosure the yield of terephthalic acid is increased by the method comprising adding a base to a reaction mixture to maintain pH at 9.0 or more.

In one embodiment, the method comprises, after depolymerizing a polyester with an enzyme, adding a base to the reaction mixture after completion of the enzymatic reaction to maintain pH at 9.0 or more.

In one embodiment, the method comprises the following steps a) and b):
a) depolymerizing a polyester comprising terephthalic acid as a monomer with an enzyme while maintaining the pH in a reactor at 5 to 9; and
b) adding a base to the reaction mixture after completion of the enzymatic reaction of step a) to maintain pH at 9.0 or more.

In one embodiment, the method comprises the following steps a) to c):
a) depolymerizing a polyester comprising terephthalic acid as a monomer with an enzyme;
b) after completion of the enzymatic reaction of step a), adding a base to the reaction mixture in which the enzymatic reaction has been completed to maintain pH at 9.0 or more; and
c) recovering terephthalic acid from the solution obtained in step b).

In any one of the foregoing embodiments, the method comprises the following steps a) and b):
a) depolymerizing polyethylene terephthalate (PET) with an enzyme while maintaining the pH in a reactor at 5 to 9; and
b) adding a base to the reaction mixture after completion of the enzymatic reaction of step a) to maintain pH at 9.0 or more.

In any one of the foregoing embodiments, the method comprises the following steps a) to c):
a) depolymerizing polyethylene terephthalate (PET) with an enzyme;
b) after completion of the enzymatic reaction of step a), adding a base to the reaction mixture in which the enzymatic reaction has been completed to maintain pH at 9.0 or more; and
c) recovering terephthalic acid from the solution obtained in step b).

In any one of the foregoing embodiments, the reaction of step a) is performed while maintaining the pH in the reactor at 5 to 9.

In one example, the pH in the reactor may be less than 9.

In one example, the pH in the reactor may be about 5 or more and less than 9.

In one example, the pH in the reactor may be about 5.2 to 9, 5.4 to 8.95, 5.6 to 8.9, 5.8 to 8.85, 6 to 8.8, 6.2 to 8.75, 6.4 to 8.7, 6.6 to 8.65, 6.8 to 8.6, 7 to 8.55, 7.1 to 8.5, 7.2 to 8.45, 7.3 to 8.4, 7.4 to 8.35, 7.5 to 8.3, 7.6 to 8.25, 7.7 to 8.2, 7.8 to 8.15, 7.9 to 8.1, or 8 to 8.05.

In any one of the foregoing embodiments, the enzymatic reaction of step a) is performed under a temperature condition of about 20°C to 80°C. For example, the enzymatic reaction may be performed under a condition of about 30°C to 70°C or about 40°C to 60°C.

In any one of the foregoing embodiments, the enzyme of step a) has PETase activity at pH 5 to 9.

In any one of the foregoing embodiments, the enzyme of step a) consists of the amino acid sequence of SEQ ID NO: 1, or comprises an amino acid sequence having at least 70% or more, 80%, 85%, 90%, 95%, 99% or more, or 100% homology or identity thereto, consists essentially of the amino acid sequence, or consists of the amino acid sequence.

With regard to the PETase, the contents disclosed in US 2020-0048621 A1 and "Three-directional engineering of IsPETase with enhanced protein yield, activity, and durability", Journal of Hazardous Materials, Volume 459, 2023, 132297, ISSN 0304-3894 are incorporated herein by reference in their entirety. In one example, the enzyme may be Z1-PETase.

Meanwhile, in the present disclosure, with regard to amino acid sequences, even when a polypeptide is described as a polypeptide "comprising" an amino acid sequence represented by a specific SEQ ID NO, a polypeptide "consisting of" an amino acid sequence represented by a specific SEQ ID NO, or a polypeptide or protein "having" an amino acid sequence represented by a specific SEQ ID NO, it is obvious that a protein having an amino acid sequence in which some sequences are deleted, modified, substituted, conservatively substituted, or added may also be used in the present disclosure, as long as the protein has the same or corresponding activity as a polypeptide consisting of the amino acid sequence of the corresponding SEQ ID NO. For example, the protein may have sequence addition that does not change the function of the protein at the N-terminus and/or C-terminus of the amino acid sequence, a naturally occurring mutation, a silent mutation thereof, or a conservative substitution, but is not limited thereto.

For example, the PETase may comprise the amino acid sequence of Z1-PETase or an amino acid sequence having at least 70% or more, 80%, 85%, 90%, 95%, 99% or more, or 100% homology or identity thereto. Meanwhile, in one example, in the sequence of the PETase of the present disclosure, the amino acid corresponding to position 121 from the N-terminus of the PETase sequence derived from *Ideonella sakaiensis* (IsPETase) may be glutamic acid, the amino acid corresponding to position 186 may be histidine, the amino acid corresponding to position 242 may be threonine, the amino acid corresponding to position 246 may be aspartic acid, the amino acid corresponding to position 233 may be cysteine, the amino acid corresponding to position 282 may be cysteine, the amino acid corresponding to position 181 may be valine, the amino acid corresponding to position 180 may be valine, the amino acid corresponding to position 37 may be aspartic acid, the amino acid corresponding to position 132 may be glutamic acid, the amino acid corresponding to position 224 may be glutamic acid, the amino acid corresponding to position 171 may be cysteine, and the amino acid corresponding to position 193 may be cysteine.

In any one of the foregoing embodiments, the enzyme is used in an amount of about 0.01% to 5% (w/w) relative to the polyester medium.

As used herein, the term "about" may be presented before a specific numerical value. As used herein, the term "about" includes not only the exact number described after the term, but also a range that is nearly the number or close to the number. Whether a number is close to or nearly the specific number recited may be determined in consideration of the context in which the number is presented. In one example, the term "about" may refer to a range of -10% to +10% of a numerical value. In another example, the term "about" may refer to a range of - 5% to +5% of a given numerical value. However, the term is not limited thereto.

In any one of the foregoing embodiments, the method comprises a solid-liquid separation of the reaction mixture after step a) and before step b).

The solid-liquid separation method is not particularly limited, and a method known in the art may be used.

In one example, the method may comprise a solid-liquid separation of the reaction mixture by centrifuging the reaction mixture after step a) and before step b).

In one example, the enzyme used in step a) may be removed in the solid-liquid separation.

In any one of the foregoing embodiments, the method comprises decolorization of the reaction mixture after step a) and before step b).

The decolorization method may comprise solvent treatment, an adsorption process, and the like, but is not particularly limited, and a method known in the art may be used.

In any one of the foregoing embodiments, the reaction mixture of step b) is obtained by further comprising physical removal of the enzyme after step a) or inactivation of the enzyme.

In any one of the foregoing embodiments, in the step b) of adding a base to the reaction mixture to maintain pH at 9.0 or more, the pH may be maintained at a pH of more than 9.

The upper limit of the pH is not particularly limited, but may be, for example, about 13 or less, 12.5, 12, 11.5, or 11 or less.

In any one of the foregoing embodiments, the base of step b) is selected from NaOH, ammonia, Ca(OH)₂, and KOH.

The ammonia may be introduced in a gaseous state or an aqueous solution state, but is not limited thereto. When ammonia gas is introduced, the ammonia may dissolve in water present in the reactor to become an aqueous solution state.

In any one of the foregoing embodiments, step b) is performed under a temperature condition of about 25°C to 100°C. In one example, the temperature condition may be about 25°C to 95°C, 25°C to 90°C, 25°C to 85°C, 30°C to 80°C, 30°C to 75°C, 30°C to 70°C, 35°C to 65°C, 35°C to 60°C, 35°C to 55°C, 40°C to 100°C, 40°C to 95°C, 40°C to 90°C, 40°C to 85°C, 40°C to 80°C, 45°C to 75°C, 45°C to 70°C, 45°C to 65°C, 45°C to 60°C, or 45°C to 55°C.

In any one of the foregoing embodiments, step b) is performed for 30 minutes or more.

The performance time of step b) may be appropriately adjusted within a range in which the recovery rate of terephthalic acid can be increased. In one example, step b) may be performed for 30 minutes or more, 1 hour or more, 1.5 hours or more, or 2 hours or more.

In any one of the foregoing embodiments, step c) may comprise crystallizing terephthalic acid by adding an acid to the solution obtained from step b) to adjust pH to 3 or less.

The acid is not particularly limited, but may be selected from H₂SO₄, HCl, H₃PO₄, and NaNO₃.

In any one of the foregoing embodiments, the pH of step c) may be appropriately adjusted within a range in which terephthalic acid can be precipitated. In one example, the pH may be 1 to 2.

In any one of the foregoing embodiments, the method further comprises a filtration of a solution containing terephthalic acid after step c). A filtration cutoff may be applied by those skilled in the art. Terephthalic acid is recovered from the solution that has undergone the filtration step, and the remaining retentate may be recycled to the reactor so that residual terephthalic acid can be precipitated.

In any one of the foregoing embodiments, a solution comprising a salt of terephthalic acid may undergo a concentration step in which water contained in the solution is removed and precipitation of terephthalic acid can be induced.

### [Mode for Carrying Out the Invention]

Hereinafter, the present disclosure will be described in more detail through Examples and Experimental Examples. However, these Examples and Experimental Examples are provided to illustrate the present disclosure, and the scope of the present disclosure is not limited to these Examples and Experimental Examples.

### Example 1. Degradation of PET Through Enzymatic Reaction

After 2,000 g of water was introduced into a 3 L reactor, 80 g of waste PET and 0.08 g of a PETase were introduced. The weight ratio of water:waste PET:PETase was 1:0.04:0.001. The PETase used was Z1-PETase described in "Three-directional engineering of IsPETase with enhanced protein yield, activity, and durability", Journal of Hazardous Materials, Volume 459, 2023, 132297, ISSN 0304-3894.

The reaction temperature was set to 50°C and the pH was set to 8. The mixture was continuously stirred for 48 hours while maintaining the pH by introducing ammonia gas.

Unreacted materials were removed from the reaction solution obtained by the reaction using a centrifuge. When the unreacted waste PET was washed with sufficient water and dried, the weight was as follows.

**[TABLE 1]**

| | Control | Experimental Example 1 | Experimental Example 2 |
|---|---|---|---|
| Weight of unreacted waste PET (g) | 22.5 | 21 | 21 |

### Example 2. Increase in Recovery Rate of Terephthalic Acid (TPA) Through pH Adjustment

In Example 1, the pH of the reaction solution remaining after removing the unreacted waste PET using a centrifuge was adjusted.

The substances added to the filtered reaction mixture during pH adjustment and the adjusted pH were as follows, and the temperature of the pH adjustment step was set to 60°C and maintained for 2 hours.

**[TABLE 2]**

| | Control | Experimental Example 1 | Experimental Example 2 |
|---|---|---|---|
| pH | No pH adjustment | 9.3 | 9.3 |
| Substance used | | Aqueous ammonia | NaOH |

After 10 g of activated carbon was added to the reaction mixture, the reaction mixture was stirred at 50°C for 3 hours and then filtered. Sulfuric acid was slowly added into the filtrate to adjust the pH to 2.0, thereby precipitating crystals.

The crystals were vacuum-filtered, washed with 300 g of water, and then dried at 60°C.

The moisture content of the filtered crystals before drying and the amount of TPA obtained after drying are shown in the table below.

**[TABLE 3]**

| | Control | Experimental Example 1 | Experimental Example 2 |
|---|---|---|---|
| Moisture content of crystals before drying (%) | 56 | 56 | 56 |
| TPA obtained after drying (g) | 43.2 | 48.2 | 48.2 |
| Content of TPA obtained after drying (%)* | 82.5 | 99.5 | 99.5 |

| | | | |
|---|---|---|---|
| * Content of TPA obtained after drying (%) = pure TPA excluding impurities after drying (g)/TPA obtained after drying (g) | | | |

From the above description, those skilled in the art to which the present disclosure pertains will understand that the present disclosure can be embodied in other specific forms without changing the technical spirit or essential features thereof. In this regard, the Examples described above should be understood as being illustrative in all respects and not restrictive. The scope of the present disclosure should be construed such that all changes or modifications derived from the meaning and scope of the claims described below and equivalent concepts thereof, rather than the detailed description above, are included in the scope of the present disclosure.

## Claims

1. A method for preparing terephthalic acid, comprising:
a) depolymerizing a polyester comprising terephthalic acid as a monomer with an enzyme while maintaining the pH in a reactor at 5 to 9;
b) after completion of the enzymatic reaction of step a), adding a base to the reaction mixture to maintain pH at 9.0 or more; and
c) recovering terephthalic acid from the solution obtained in step b).

2. The method according to claim 1, wherein the base in step b) is selected from NaOH, ammonia, Ca(OH)₂, and KOH.

3. The method according to claim 1, wherein step b) is performed at a temperature of 25°C to 100°C.

4. The method according to claim 1, wherein step b) is performed for 30 minutes or more.

5. The method according to claim 1, comprising a solid-liquid separation of the reaction mixture after step a) and before step b).

6. The method according to claim 5, wherein the solid-liquid separation is centrifuging the reaction mixture.

7. The method according to claim 1, comprising a step of decolorizing the reaction mixture after step a) and before step b).

8. The method according to claim 1, wherein the enzyme of step a) has PETase activity at pH 5 to 9.

9. The method according to claim 1, wherein the polyester is selected from polyethylene terephthalate (PET), polyethylene terephthalate glycol (PETG), polyethylene co-isosorbide terephthalate (PEIT), polytrimethylene terephthalate (PTT), polybutylene adipate terephthalate (PBAT), polycyclohexylenedimethylene terephthalate (PCT), and polybutylene terephthalate (PBT).

10. The method according to claim 1, wherein step c) comprises crystallizing terephthalic acid by adding an acid to the solution obtained from step b) to adjust pH to 3 or less.

11. The method according to claim 10, wherein the acid is selected from H₂SO₄, HCl, H₃PO₄, and NaNO₃.
